Europäisches Patentamt

**European Patent Office**

**Office européen des brevets**

(11) Veröffentlichungsnummer: **0 158 683**
**B1**

(12) **EUROPÄISCHE PATENTSCHRIFT**

(45) Veröffentlichungstag der Patentschrift:
**05.10.88**

(21) Anmeldenummer: **84104242.7**

(22) Anmeldetag: **14.04.84**

(51) Int. Cl.⁴: **A 61 M 5/14**

(54) Vorrichtung zum intermittierenden Applizieren flüssiger Arzneimittel.

(43) Veröffentlichungstag der Anmeldung:
**23.10.85 Patentblatt 85/43**

(45) Bekanntmachung des Hinweises auf die Patenterteilung:
**05.10.88 Patentblatt 88/40**

(84) Benannte Vertragsstaaten:
**AT BE CH DE FR GB IT LI NL SE**

(56) Entgegenhaltungen:
**EP - A - 0 039 044**
**CH - A - 610 763**
**DE - A - 3 307 810**
**FR - A - 2 384 134**
**FR - A - 2 387 046**

(73) Patentinhaber: **Ferring Biotechnik GmbH,**
**Wittland 11-13, D-2300 Kiel 1 (DE)**

(72) Erfinder: **Kölln, Harm, Fallsreep 43, D-2300 Kiel 17 (DE)**

(74) Vertreter: **Türk, Gille, Hrabal, Bruckner Strasse 20,**
**D-4000 Düsseldorf 13 (DE)**

**Beschreibung**

Die Erfindung betrifft eine Vorrichtung zum intermittierenden pulsatorischen Applizieren flüssiger Arzneimittel, mit einem auswechselbaren Vorratsbehälter für das Arzneimittel, einem an diesem angebrachten flexiblen Schlauch, einem am äusseren Ende des Schlauches anschliessbaren Katheter, einer auf den Schlauch einwirkenden Rollenpumpe, einem Antriebsmotor für die Rollenpumpe und einem Zeitgeber für Förderimpuls- und Pausenzeiten der Rollenpumpe.

Bei einer bekannten Vorrichtung dieser Gattung (EP-A 0 039 044) ist die Steuerung des Betriebsablaufes noch ungenügend. Es können zwar einfache Programmabläufe eingestellt werden, wozu ein Pausen- und Applikationszeiten messender Zeitgeber mit einem Taktgenerator, einem Frequenzteiler und einer Treiberstufe vorgesehen ist. Die Einstellung eines Programmes ist aber umständlich und letztlich zu ungenau. Hinzu kommt, dass Fehler im Programmablauf, beispielsweise wenn sich ein Schlauch verklemmt oder ein Treibertransistor durchbrennt, nicht auffallen, so dass die Gefahr der Applikation ungenügender oder zu grosser Mengen Arzneimittel besteht.

Es ist ferner eine Flüssigkeitspumpe zum Applizieren flüssiger Arzneimittel bekannt (FR-A 2 384 134), mit der aus einem von der Pumpe getrennten Vorratsbehälter flüssige Arzneimittel einem Katheter zugeführt werden können. Dabei ist zwar eine Flussrate und eine Arbeitsdauer einstellbar, jedoch ist eine langfristig vorher programmierbare intermittierende pulsatorische Förderung des Arzneimittels nicht vorgesehen. Eine derartige Vorrichtung eignet sich weder für ein tragbares Gerät noch zum Applizieren flüssiger Arzneimittel in sehr kleinen Mengen über einen längeren Zeitraum von auch mehreren Tagen.

Der Erfindung liegt die Aufgabe zugrunde, die aus der EP-A 0 039 044 bekannte Vorrichtung derart zu verbessern, dass sie einfacher und sicherer auf die Bedürfnisse der jeweiligen Patientin eingestellt werden kann und Fehler im Programmablauf weitgehend vermieden werden können, sich aber, wenn sie doch einmal auftreten sollten, sofort feststellen und dann einfach und problemlos beheben lassen.

Diese Aufgabe wird bei einer Vorrichtung der eingangs genannten Gattung mit den kennzeichnenden Merkmalen des Anspruchs 1 gelöst.

Mit dieser Vorrichtung kann der Anwender, insbesondere der Arzt, ein auf jeden Patienten speziell abgestimmtes Behandlungsprogramm aufstellen und auch für lange Behandlungszeiten einspeichern. Beim Programmieren kann in Feldern gearbeitet werden. Die möglichen Programmschritte lassen sich beliebig aufteilen. Dementsprechend muss man dem Mikrocomputer nicht von vornherein aufgeben, was programmiert werden soll. Vielmehr ist das Betriebsprogramm durch die Wahlmöglichkeit zwischen mehreren Feldern sozusagen vorprogrammiert.

Nach einem weiteren Merkmal der Erfindung kann an den Mikrocomputer ein Drehzahlmesser für den Antriebsmotor angeschlossen sein. Somit lässt sich die Drehzahl des Motors und damit der Rollenpumpe überwachen, so dass Fehler im Programmablauf sofort festgestellt werden und die Möglichkeit der Abhilfe gegeben ist.

Nach einem anderen Merkmal der Erfindung ist die Vorrichtung mit einer Anzeige versehen, mit der die eingegebenen Daten kontrolliert werden können und die ausserdem dazu benutzt werden kann, die aufgezeigten Fehlersituationen genauer zu bezeichnen, d.h. ob der Motor ungenau läuft, der Arzneimittelvorrat erschöpft ist und dergleichen. Zusätzlich kann an den Mikrocomputer ein Signalgeber zum Anzeigen von Störsituationen oder Fehlersituationen angeschlossen sein, beispielsweise ein akustischer Signalgeber. Die Störsituation bzw. der Fehler kann nach Ertönen eines Signaltones von der Azeige abgelesen werden, so dass schnell Abhilfe zu schaffen ist.

Ein weiteres zusätzliches Merkmal der Erfindung sieht vor, dass eine elektronische Sicherung den Antriebsmotor für die Rollenpumpe ausschaltet, falls ein Element des Mikrocomputer-Systems defekt ist, beispielsweise die Uhr, die Kalenderanzeige oder der Mikrocomputer selbst, und dementsprechend der Antrieb für die Rollenpumpe und dieselbe unkontrolliert arbeitet.

Als Energiequelle ist nach einem weiteren Merkmal der Erfindung eine 9-Volt-Blockbatterie vorgesehen. Ausserdem ist zur Datensicherung während eines Batteriewechsels oder, wenn die Antriebsbatterie erschöpft ist, eine 3-Volt-Knopfzelle vorhanden, die, da sie kaum benutzt wird, eine verhältnismässig lange Lebensdauer hat.

Jedem Tastenfeld sind erfindungsgemäss mehrere Programmierfelder zugeordnet, so dass das jeweilige Programm bzw. dessen Ablauf individuell eingestellt werden kann. Ein Programmierfeld dient beispielsweise zum Einstellen der Pausenzeiten und ein weiteres Programmierfeld zum Einstellen der Förderzeiten. Jeder Steuerfunktion des Mikrocomputers ist dementsprechend ein eigenes Programmierfeld zugeordnet. Einzelheiten über das Programmieren sind weiter unten erläutert.

Da die erfindungsgemässe Vorrichtung mit einem Signalgeber zum Anzeigen von Störungen oder Fehlern des Programmablaufes versehen ist, lässt sich die Vorrichtung mit grosser Betriebssicherheit benutzen. So würde beispielsweise ein programmwidriger Lauf des Motors, weil sich ein Schlauch verklemmt hat und der Motor dementsprechend langsamer liefe bzw. ganz stehenbliebe, sofort angezeigt werden, ebenso ein zu schneller Lauf des Motors aufgrund des Durchbrennens eines Treibertransistors und der dadurch bedingten zu grossen Fördermenge des Arzneimittels. Weiterhin kann der Signalgeber auch dazu benutzt werden, um anzuzeigen, wenn eine vorher bestimmte Menge Injektionsflüssigkeit bzw. Arzneimittel verbraucht ist. Zu diesem Zweck muss der Mikrocomputer oder Mikroprozessor aufaddieren, wieviel Flüssigkeit während der einzelnen Förderperioden verbraucht wurde. Diese Daten kann man jederzeit abrufen und mittels der Anzeige sichtbar machen, so dass die verbrauchte Menge des Arzneimittels jederzeit überprüft werden kann, d.h. also auch bevor der Signalgeber anzeigt, dass der Arzneimittelvorrat bald erschöpft sein wird.

Die Art der Programmierung ist deshalb besonders zweckmässig, weil der Anwender, beispielsweise der Arzt, mit einzelnen Feldern arbeiten kann. Daher kann er die möglichen Programmierschritte beliebig aufteilen und muss nicht von vornherein dem Microcomputer aufgeben, was er programmieren möchte. Vielmehr ist das Betriebsprogramm durch die Wahlmöglichkeit zwischen mehreren Feldern sozusagen vorprogrammierbar.

Es sind die verschiedensten Programme möglich. Beispielsweise kann ein Programm mit festem Zyklusschema eingestellt werden, jedoch sind auch tägliche Änderungen des Zyklusschemas programmierbar, beispielsweise wenn nachts oder während Ruhezeiten andere Pausenzeiten notwendig sind. Dabei kann man auch täglich mehrere Änderungen des Programms einstellen, so dass auch Ruhezeiten beispielsweise am frühen Nachmittag berücksichtigt werden können.

Dementsprechend lässt sich mit der erfindungsgemässen Vorrichtung die Verabreichung flüssiger Arzneimittel sehr genau an die täglichen Bedürfnisse des Patienten anpassen. Dadurch erhält man eine viel genauere Therapiemöglichkeit.

Soll zu einem bestimmten Zeitpunkt ein vollständig anderes Programm ablaufen, können über ein weiteres Feld Datum und Uhrzeit für den Beginn dieses Programms eingegeben werden. Dabei lassen sich auch mehrere Zeitpunkte für vollständige Änderungen des Programms eingeben. Die Programmierschritte werden vom Mikrocomputer in Speicherplätze abgelegt. So stehen beispielsweise etwa 300 Speicherplätze zur Verfügung, die beliebig über Programmierfelder verteilt werden können.

Es ist auch möglich, einen Bolus zu programmieren, der bei Bedarf vom Arzt oder vom Patienten abgerufen werden kann. Unter einem Bolus versteht man die Möglichkeit, durch Knopfdruck eine zusätzliche Arzneimittelgabe zu veranlassen. Ein Bolus ist beispielsweise bei nicht vorprogrammierbarem erhöhten Bedarf des Medikamentes, zum Beispiel nach besonderer körperlicher Belastung oder dergleichen, erforderlich. Aus Sicherheitsgründen lässt sich eine Mindestpause zwischen zwei aufeinanderfolgenden Boluszugaben einstellen.

Mit der computergesteuerten erfindungsgemässen Vorrichtung ist es auch möglich, jeden Pumpenschlauch auf sein Fördervolumen zu eichen. Zu diesem Zweck wird ein Messvolumen gefördert. Der Anwender betätigt zu Beginn und zum Ende der Förderung eine Taste. Der Mikrocomputer berechnet dann selbständig die notwendige Drehgeschwindigkeit des Motors für ein gewünschtes Fördervolumen der Pumpe pro Minute. Diese Möglichkeit ist für besondere Medikamente von Bedeutung, bei deren Anwendung es auf sehr kleine bzw. kleinste Bruchteilsmengen ankommt.

In der Zeichnung ist ein Ausführungsbeispiel der erfindungsgemässen computergesteuerten Vorrichtung zum Applizieren flüssiger Arzneimittel dargestellt, und zwar zeigt

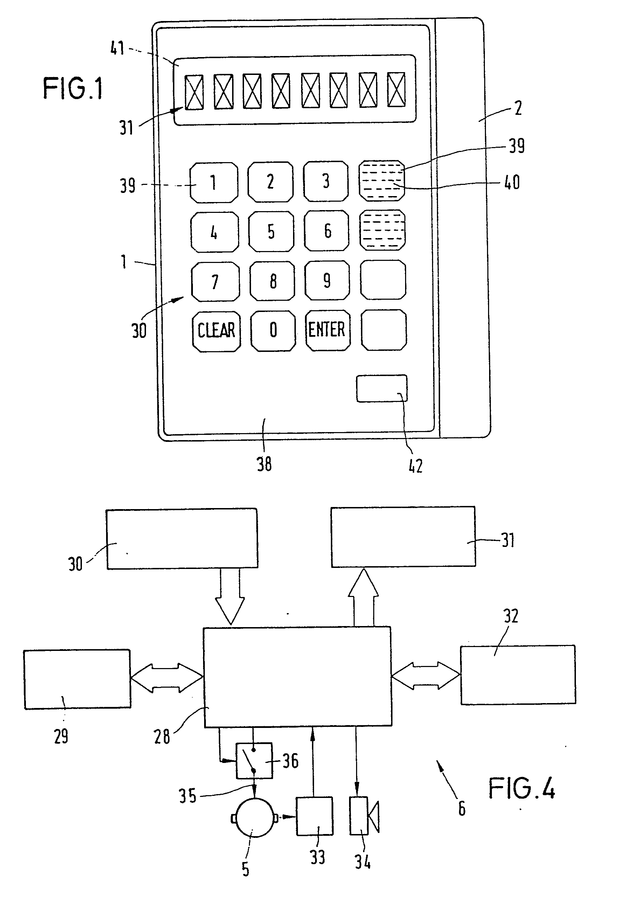

Fig. 1 eine Ansicht der Vorderseite der Vorrichtung,

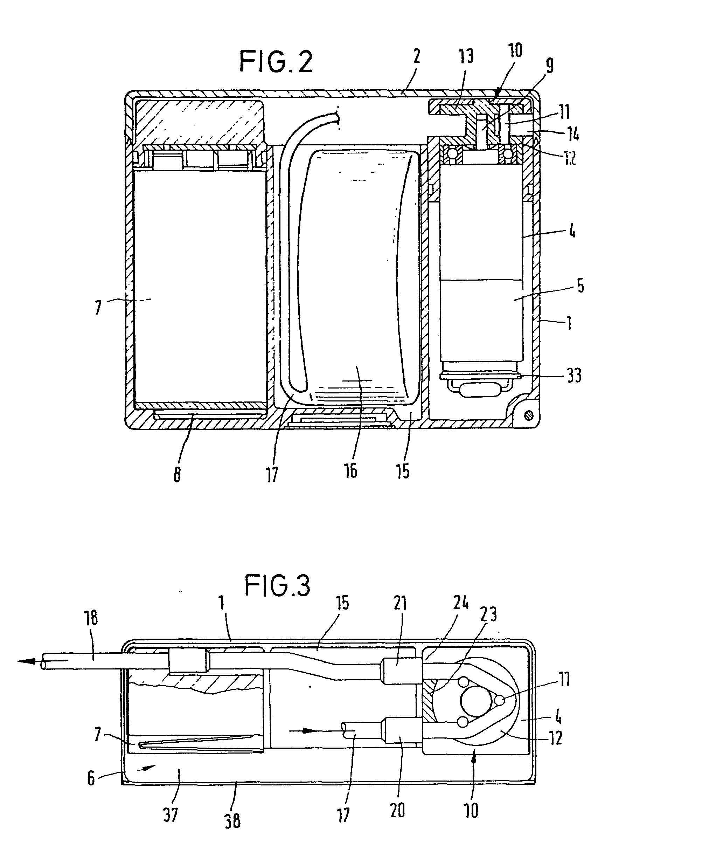

Fig. 2 einen senkrechten Längsschnitt der Vorrichtung,

Fig. 3 eine Draufsicht auf die Vorrichtung in Fig. 1 von rechts gesehen, wobei der Deckel des Gehäuses abgenommen ist, und

Fig. 4 ein Blockdiagramm der elektronischen Steuerung der Vorrichtung aus Fig. 1 bis 3.

Die dargestellte Vorrichtung hat ein kastenartiges Gehäuse 1 mit abnehmbar aufgestecktem Deckel 2, so dass die wichtigsten Teile der Vorrichtung bei Bedarf von aussen zugänglich sind.

Im Gehäuse 1 ist eine Rollenpumpe 4 mit Antriebsmotor 5 untergebracht, der über eine elektronische Steuerung 6 von einer 9-Volt-Blockbatterie 7 nach einem vorgegebenen Programm angetrieben wird. Die Blockbatterie 7 ist auswechselbar im Gehäuse 1 untergebracht. Eine weitere Batterie, nämlich eine 3-Volt-Knopfzelle 8, dient zur Datensicherung der elektronischen Steuerung 6, wenn die Blockbatterie 7 erschöpft sein sollte und ausgewechselt wird. Auch die Knopfzelle 8 ist auswechselbar im Gehäuse 1 untergebracht.

Auf der Abtriebswelle 9 des Motors 5 der Rollenpumpe 4 ist ein Kopf 10 drehfest angebracht, der mit achsparallel zur Abtriebswelle 9 liegenden Nadelrollen 11 versehen ist, die sich zwischen einer dicken Scheibe 12 und einer dünnen Abdeckscheibe 13 erstrecken, so dass sie in einer Art Führungsschlitz 14 des Kopfes 10 liegen.

In einer Ausnehmung 15 im Gehäuse 1 steckt auswechselbar ein in seiner Grösse konfektionierter flexibler Vorratsbehälter 16 für flüssiges Arzneimittel bzw. Injektionslösung, an den als Auslass ein Schlauch 17 angeschlossen ist, der durch den Schlitz 14 des Kopfes 10 der Rollenpumpe 4 geführt und dabei fest auf die Nadelrollen 11 gespannt ist. Dieser Schlauch 17 endet in einem Anschlussstück 18, an das ein Katheter auswechselbar angesetzt werden kann. Zum Festspannen des Schlauches 17 am Kopf 10 der Rollenpumpe 4 sind auf diesem Muffen 20 und 21 befestigt, die sich hinter eine im Gehäuse 1 vorgesehene hochstehende Wand 23 legen, welche seitliche Schlitze 24 vom Einlegen und Herausnehmen des Schlauches 17 enthält.

Der Motor 5 der Rollenpumpe 4 wird mit Gleichstrom angetrieben, welcher von der Blockbatterie 7 entsprechend einem von der elektronischen Steuerung 6 bestimmten Programm geliefert wird. Durch Eingeben einer Ziffernkombination kann der Motor 5 unabhängig vom vorgegebenen Programm eingeschaltet werden. Mit einer anderen Ziffernkombination kann man abhängig vom Programm der Steuerung 6 eine Bolusgabe vornehmen.

Die in einen scheibenförmigen Kunststoff-Block 37 (Fig. 3) eingegossene elektronische Steuerung 6 hat, wie Fig. 4 zeigt, als Kernelement einen Mikrocomputer 28 mit an diesen angeschlossenem Programmspeicher 29. Die Programmierung ist mittels eines Tastenfeldes 30 vorzunehmen, das sich in einer auf die Vorderseite des Kunststoff-Blockes 37 aufgeklebten flexiblen Platte 38 befindet. Das Tastenfeld 30 hat insgesamt 16 in die Platte 38 integrierte Tasten 39, auf deren Rückseite sich streifenförmige Leiter 40 befinden, welche als Kontakte zur elektronischen Steuerung 6 dienen. Durch Drücken der einzelnen Tasten 39 können entsprechende Daten in den Programmspeicher 29 des Mikrocomputers 28 eingegeben werden. Hinter einem Sichtfeld 41 der Platte 38 befindet sich als Teil der elektronischen Steuerung 6 eine Anzeige 31, die hier eine in den Block 37 eingegossene Flüssigkeitskristallanzeigetafel ist. Hinter einem weiteren Sichtfeld 42 der Platte 38 ist die Seriennummer des Gerätes auf dem Block 37 angebracht.

Der Mikrocomputer 28 vergleicht ständig die Daten des Programmspeichers 29 mit Uhrzeit und Datum einer Kalender-Uhr 32, die eine Quarzuhr ist.

An den Mikrocomputer 28 ist der Motor 5 der Rollenpumpe 4 angeschlossen. Der Motor 5 wird entsprechend dem eingegebenen Programm vom Mikrocomputer 28 angesteuert. Die Drehzahl des Motors 5 wird von einem Drehzahlmesser 33 an den Mikrocomputer 28 gemeldet und, falls erforderlich, korrigiert. Ein Signalgeber 34, im vorliegenden Falle ein akkustischer Signalgeber, ertönt, wenn die Batterie 7 ausgewechselt werden muss, wenn der Motor 5 programmwidrig läuft und wenn eine vorbestimmte Menge flüssiges Arzneimittel bzw. Injektionsflüssigkeit verbraucht ist. Ertönt der Signalgeber 34, kann an der Anzeige 31 der Grund dafür abgelesen werden.

Die als Sicherung vorgesehene Knopfzelle 8 sichert beim Auswechseln der Blockbatterie 7 das eingegebene Programm und sorgt dafür, dass die Kalender-Uhr 32 ohne Unterbrechung weiterläuft. Der Mikrocomputer 28 steigt daher nach dem Auswechseln der Batterie 7 zeitrichtig wieder in das eingegebene Programm ein. Daher kann die Batterie 7 auch während der Anwendung und vom Patienten selbst ausgetauscht werden.

Die elektronische Steuerung 6 bzw. deren Elektrik sowie die Batterien 7 und 8 sind wasserdicht gekapselt. Somit kann weder Arzneimittellösung aus einem undicht gewordenen Vorratsbehälter 16 noch Wasser von aussen an spannungsführende Teile gelangen.

Das Standardzyklusschema des Programms der Vorrichtung sieht eine Pausenzeit (z.B. 89 Minuten), eine Laufzeit (z.B. 1 Minute) und ein Pumpvolumen (z.B. 50 µl/min.) vor. Zum Programmieren gibt eine programmberechtigte Person, beispielsweise der Arzt, einen Code über das Tastenfeld 30 ein, wodurch der Mikrocomputer 28 auf Programmierbereitschaft geschaltet wird. Über die Tasten des Tastenfeldes 30 können verschiedene Programmierfelder ausgewählt werden. Die Anzeige 31 zeigt dann jeweils die in das betreffende Programmierfeld eingegebenen Daten an.

Beispielsweise erscheint bei Wahl des Feldes «50» auf der Anzeige 31 die eingestellte Pausenzeit. Diese Zeit kann mit Stunden, Minuten und Sekunden neu eingestellt bzw. eingespeichert werden. Bei Wahl des Feldes «51» ist in gleicher Weise die Laufzeit der Rollenpumpe bzw. deren Förderzeit einprogrammierbar. Über das Feld «52» lässt sich das Pumpenvolumen pro Minute für die jeweiligen Förderzeiten oder Laufzeiten programmieren.

Ohne weitere Programmierung wird dieses Zyklusschema wiederholt. Sind Abweichungen von diesem Zyklusschema gewünscht, beispielsweise wenn nachts oder während Ruhezeiten andere Pausenzeiten notwendig sind, so kann man entsprechende Daten über ein weiteres Feld «53» einprogrammieren. Dazu wird im Feld «53» die Uhrzeit eingegeben, zu der die Abweichung vom Zyklusschema erfolgen soll. Über die Felder «50» bis «52» werden dann die Änderungen programmiert. Dabei ist es möglich, mehrere tägliche Änderungen oder Abweichungen vom Zyklusschema vorzusehen.

Soll ab einem bestimmten Zeitpunkt ein vollständig anderes Programm ablaufen, können über ein weiteres Feld «54» Datum und Uhrzeit dieses Zeitpunktes eingegeben werden. In vorstehend beschriebener Weise kann dann der tägliche Ablauf des Programms neu bestimmt werden. Auch in diesem Falle lassen sich mehrere Zeitpunkte für vollständige Programmänderungen einspeichern. Die Programmierdaten werden vom Mikrocomputer 28 in den Programmspeicher 29 eingegeben und dort gespeichert. Beispielsweise stehen 300 Speicherplätze zur Verfügung, die beliebig von allen Feldern «50» bis «54» in Anspruch genommen werden können.

Das Feld «50» usw. wird eingegeben, indem man nacheinander die Tasten «5» und «0» drückt. Bei der Eingabe anderer Felder verfährt man entsprechend.

Fig. 4 zeigt ferner, dass in den Stromkreis 35 des Motors 5 eine Sicherung 36 eingebaut ist, die eine sich immer wieder nach einer vorgegebenen Zeitspanne ausschaltende elektronische Sicherung ist. Mit dieser Sicherung 36 ist der Mikrocomputer 28 verbunden. Wird die Sicherung 36 vom Mikrocomputer 28 eingeschaltet, schaltet sie sich selbsttätig nach 0,1 sec. wieder aus. Dementsprechend muss sich der Mikrocomputer 28 mindestens alle 0,1 sec. bei der Sicherung 36 mit einem «ok» melden. Bei normalem Betrieb der Vorrichtung wird die Sicherung 36 in regelmässigen Abständen vom Mikrocomputer 28 auf Funktionstüchtigkeit überprüft.

Ist ein Teil der Elektronik defekt, beispielsweise die Uhr, der Kalender oder auch der Mikrocomputer selbst, wird möglicherweise kein Alarm gegeben, so dass das Pumpensystem dann unkontrolliert arbeitet. In einem solchen Fall schaltet die elektronische Sicherung 36 den Motor 5 für die Rollenpumpe 4 sofort aus, bevor Schaden entstehen kann.

**Patentansprüche**

1. Tragbare Vorrichtung zum intermittierenden pulsatorischen Applizieren flüssiger Arzneimittel, mit einem auswechselbar in einem Gehäuse untergebrachten Vorratsbehälter (16) für das Arzneimittel, einem an dem Vorratsbehälter angebrachten flexiblen Schlauch (17), einem am äusseren Ende des Schlauches anschliessbaren Katheter, einer auf den Schlauch einwirkenden Rollenpumpe (4), einem mittels einer Batterie anzutreibenden Antriebsmotor (5) für die Rollenpumpe und einem Zeitgeber für Förderimpuls- und Pausenzeiten der Rollenpumpe, dadurch gekennzeichnet, dass sie zum Steuern des Betriebsablaufes des Antriebsmotors (5) mit einem über ein Tastenfeld (30) programmierbaren Mikrocomputer (28) mit Programmspeicher (29) ausgestattet, an den Mikrocomputer (28) eine Uhr mit Kalender (32) sowie ein Drehzahlmesser (33) für den Antriebsmotor (5) und ein Signalgeber (34) zum Anzeigen von Störsituationen angeschlossen und eine Anzeige (31) zum Kontrollieren der eingegebenen Daten und Anzeigen von Störsituationen vorgesehen ist, wobei dem Tastenfeld (30) mehrere Programmierfelder (50 bis 54) mit jeweils einem Programmierfeld für jede Steuerfunktion des Mikrocomputers (28) zugeordnet sind.

2. Vorrichtung nach Anspruch 1, dadurch gekennzeichnet, dass der Signalgeber (34) akustische Signale liefert.

3. Vorrichtung nach Anspruch 1 oder 2, dadurch gekennzeichnet, dass sie als Energiequelle eine 9-Volt-Blockbatterie (7) und zur Datensicherung während des Batteriewechsels eine 3-Volt-Knopfzelle (8) enthält.

4. Vorrichtung nach einem der Ansprüche 1 bis 3, dadurch gekennzeichnet, dass sie mit einer in Notfällen den Antriebsmotor (5) für die Rollenpumpe (4) ausschaltenden Sicherung (36) versehen ist.

5. Vorrichtung nach Anspruch 4, dadurch gekennzeichnet, dass die Sicherung (36) eine in den Stromkreis (35) des Antriebsmotors (5) eingeschaltete, sich stets nach einer vorgegebenen Zeitspanne ausschaltende elektronische Sicherung ist.

6. Vorrichtung nach Anspruch 5, dadurch gekennzeichnet, dass der Mikrocomputer (28) mit der Sicherung (36) verbunden ist und diese immer wieder einschaltet.

**Claims**

1. A portable device for the intermittent pulsatory application of liquid pharmaceuticals, comprising a storage container (16) for the pharmaceutical being changeable mounted within a housing, a flexible tube (17) mounted at the storage container, a catheter connectable to the outside end of the tube, a rolling pump (4) acting on the tube, a drive motor (5) for the rolling pump driven by a battery and a timer for delivery impulse and pause times of the rolling pump, characterized in that a micro-computer (28) having a programm storage (29) is provided which is programmable by a keyboard (30) for controlling the operation of the drive motor is provided, a clock with calendar (32) and a tachometer (33) for the drive motor (5) and a signal generator (34) to indicate disturbances are connected to the micro-computer (28), and a display (31) for controlling the entered data and displaying the disturbances is provided, whereby several program fields (50 to 54) having a program field for each control function of the micro-computer (28) are co-ordinated to the keyboard (30).

2. A device according to claim 1, characterized in that the signal generator (34) delivers acoustic signals.

3. Device according to claim 1 or 2, characterized in that it comprise as energy source a 9-volt block battery (7) and for securing data during the battery change a 3-volt button cell (8).

4. Device according to one of the claims 1 to 3, characterized in that a safety device (36) is provided which switches off the drive motor (5) for the rolling pump (4) in emergency cases.

5. A device according to claim 4, characterized in that the safety device (36) is a electronic safety device being set in the current circuit (35) of the drive motor (5) and switching off ifself always at regular intervals.

6. A device according to claim 5, characterized in that the microcomputer (28) is connected to the safety device (36) and switches it on again and again.

**Revendications**

1. Dispositif portable permettant d'administrer de façon pulsatoire et intermittente des médicaments liquides, comportant un réservoir (16) logé de façon interchangeable dans un boîtier et recevant le médicament, un tuyau flexible (17) monté sur le réservoir, un cathéter pouvant être raccordé à l'extrémité extérieure du tuyau, une pompe à rouleaux (4) agissant sur le tuyau, un moteur d'entraînement (5) destiné à être alimenté par une pile et prévu pour la lampe à rouleaux et un chronomètre pour mesurer les durées d'impulsions de refoulement et de pauses entre impulsions de la pompe à rouleaux, caractérisé en ce qu'il est équipé, pour la commande du cycle de fonctionnement du moteur d'entraînement (5), d'un micro-ordinateur (28), qui est programmable par l'intermédiaire d'un clavier (30) qui est équipé d'une mémoire de programmes (29) et auquel sont raccordés une montre munie d'un calendrier (32) ainsi qu'un tachymètre (33) pour le moteur d'entraînement (5) et un générateur de signaux (34) pour indiquer des cas de perturbations, et il est prévu un dispositif d'affichage (31) servant à contrôler les données introduites et à afficher des cas de perturbations, plusieurs zones de programmation (50 à 54) étant associées au clavier (30) et une zone de programmation étant prévue respectivement pour chaque fonction de commande du micro-ordinateur (28).

2. Dispositif selon la revendication 1, caractérisé en ce que le générateur de signaux (34) délivre des signaux acoustiques.

3. Dispositif selon la revendication 1 ou 2, caractérisé en ce qu'il contient, comme source d'énergie, une pile monobloc de 9 volts (7) et, pour assurer la sécurité des données lors du remplacement de la pile, une pile ronde à 3 volts (8).

4. Dispositif selon l'une des revendications 1 à 3, caractérisé an ce qu'il comporte un coupe-circuit (36) débranchant, dans des cas d'urgence, le moteur d'entraînement (5) prévu pour la pompe à rouleaux (3).

5. Dispositif selon la revendication 4, caractérisé en ce que le coupe-circuit (36) est un coupe-circuit électronique branché dans le circuit (35) du moteur d'entraînement (5) et se déclenchant toujours au bout d'un intervalle de temps prédéterminé.

6. Dispositif selon la revendication 5, caractérisé en ce que le micro-ordinateur (28) est relié au coupe-circuit (36) et rebranche toujours ce dernier.

0 158 683

FIG.1

FIG.4

7

# FIG.2

# FIG.3